# EUROPEAN PATENT SPECIFICATION

(11) **EP 2 583 639 B1**
(45) Date of publication and mention of the grant of the patent: **25.06.2014**
(21) Application number: 13151327.7
(22) Date of filing: 19.01.2011
(51) Int. Cl.: A61F 2/18, H04R 25/00

(54) **Incus replacement partial ossicular replacement prosthesis**
Incus Ersatz teilweise Ossicular Ersatz Prothese
Remplacement de l'enclume ossiculaire remplacement partiel de la prothèse

(30) Priority: 21.01.2010 US 296928 P; 20.07.2010 US 365824 P
(43) Date of publication of application: 24.04.2013
(62) Divisional of application: 11701185.8
(73) Proprietor: MED-EL Elektromedizinische Geraete GmbH, Innsbruck 6020 (AT)
(72) Inventor: Ball, Geoffrey, 6094 Axams (AT); Santek, Michael, 6020 Innsbruck (AT); Lenarz, Thomas, 30559 Hannover (DE)
(74) Representative: Downing, Michael Philip

(56) References cited:
- EP-A1- 2 135 584
- DE-U1-202005 015 533
- US-A1- 2007 249 890
- US-A1- 2007 255 405

## Description

### FIELD OF THE INVENTION

The present invention relates to medical implants, and more specifically to a novel ossicular prosthesis arrangement.

### BACKGROUND ART

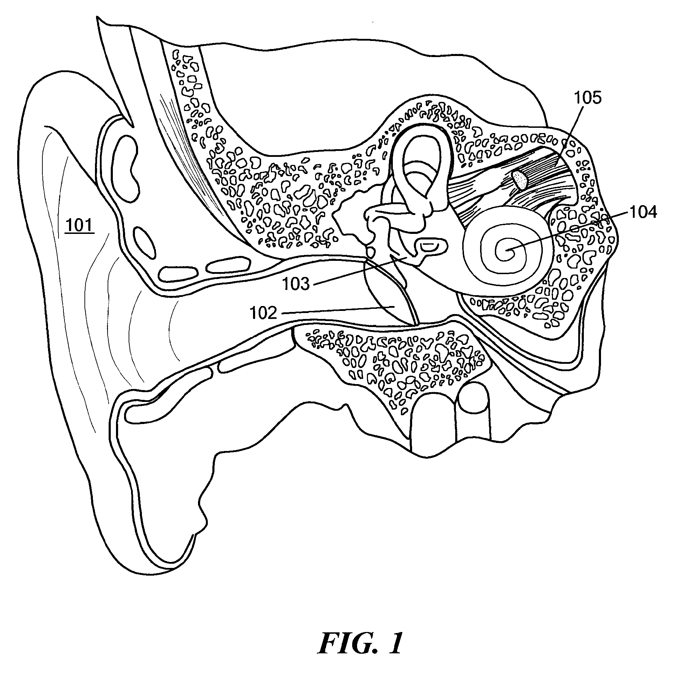

A normal ear transmits sounds as shown in Figure 1 through the outer ear **101** to the tympanic membrane (eardrum) **102.** which moves the ossicles of the middle ear **103** (malleus, incus, and stapes) that vibrate the oval window and round window openings of the cochlea **104.** The cochlea **104** is a long narrow duct wound spirally about its axis for approximately two and a half turns. It includes an upper channel known as the scala vestibuli and a lower channel known as the scala tympani, which are connected by the cochlear duct. The cochlea **104** forms an upright spiraling cone with a center called the modiolar where the spiral ganglion cells of the acoustic nerve **105** reside. In response to received sounds transmitted by the middle ear **103,** the fluid-filled cochlea **104** functions as a transducer to generate electric pulses which are transmitted to the cochlear nerve **105,** and ultimately to the brain.

Hearing is impaired when there are problems in the ability to transduce external sounds into meaningful action potentials along the neural substrate of the cochlea **104.** To improve impaired hearing, auditory prostheses have been developed. For example, when the impairment is related to operation of the middle ear **103,** a conventional hearing aid may be used to provide acoustic-mechanical stimulation to the auditory system in the form of amplified sound.

Middle ear implants also have been developed that employ electromagnetic transducers to mechanically stimulate the structures of the middle ear **103.** A coil winding is held stationary by attachment to a non-vibrating structure within the middle ear **103** and a microphone signal current is delivered to the coil winding to generate an electromagnetic field. A magnet is attached to an ossicle within the middle ear **103** so that the magnetic field of the magnet interacts with the magnetic field of the coil. The magnet vibrates in response to the interaction of the magnetic fields, causing vibration of the bones of the middle ear **103**. *See* U.S. Patent 6,190,305.

Middle ear implants using electromagnetic transducers can present some problems. Many are installed using complex surgical procedures which present the usual risks associated with major surgery and which also require disarticulating (disconnecting) one or more of the bones of the middle ear **103.** Disarticulation deprives the patient of any residual hearing he or she may have had prior to surgery, placing the patient in a worsened position if the implanted device is later found to be ineffective in improving the patient's hearing. US2007/0255405 A1 discloses a middle ear prosthesis comprising a deformable body with fingers for clamping to a bone of the middle ear and a cochlear striker.

### SUMMARY OF THE INVENTION

Embodiments of the present invention are directed to an ossicular, or middle ear, prosthesis comprising:
an elongated prosthesis member having a proximal end and a distal end;
a cochlea striker surface at the distal end of the prosthesis member for engaging an outer cochlear surface of a recipient patient; and
a transducer clamp at the proximal end of the prosthesis member including a plurality of clamping fingers,
characterised in that the clamp is a transducer clamp, its fingers being for securely engaging the outer surface of an enclosed acoustic signal transducer such that acoustic vibration of the signal transducer is coupled by the prosthesis member to the cochlear surface,
and in that the prosthesis member is formed from a single foldable plane structure.

A lockingclamp may be provided at the proximal end of the prosthesis member and includes a clamp strap having a fixed end and a free end, and a locking head at the fixed end of the clamp strap which has a strap opening for insertion of the free end of the clamp strap. The clamp strap passes around an ossicle of the middle ear (e.g., a disarticulated incus) in a closed loop and is fixedly engaged by the locking head such that acoustic vibration of the ossicle is coupled by the prosthesis member to the cochlea surface.

The cochlea surface may include the round window membrane and/or the oval window membrane of the cochlea. The locking clamp may be a cable tie-type clamp. The prosthesis member may be made of titanium. The clamp strap may be made of plastic or a polymer material.

### BRIEF DESCRIPTION OF THE DRAWINGS

Figure 1 shows various anatomical structures of a normal human ear.
Figure 2A shows an example of an ossicular prosthesis in an open position.
Figure 2B shows another example of an ossicular prosthesis in a closed position.
Figure 3A shows another example of an ossicular prosthesis in a closed position.
Figure 3B and 3C shows the ossicular prosthesis of Fig. 3A in situ in a recipient patient.
Figure 4A shows an elevated perspective view of another example of an ossicular connector for coupling an acoustic signal transducer to an ossicle in the middle ear.
Figure 4B shows the ossicular prosthesis of Fig. 4A in situ in a recipient patient.
Figure 5A-C shows perspective views of various alternative examples of an ossicular connector.
Figure 6A shows an embodiment of a single foldable plane structure for forming a middle ear prosthesis.
Figure 6B shows the structure of Fig. 6A as folded into an open middle ear prosthesis ready to be surgically attached.

### DETAILED DESCRIPTION OF SPECIFIC EMBODIMENTS

Various embodiments of the present invention are directed to an ossicular prosthesis specifically referred to as a partial ossicular replacement prosthesis (PORP). The PORP uses a locking clamp having a clamp strap to connect a prosthesis member to an ossicle so as to couple vibration from the ossicle to the outer cochlea surface of a recipient patient for otologic reconstruction thereby providing sound sensation to the patient.

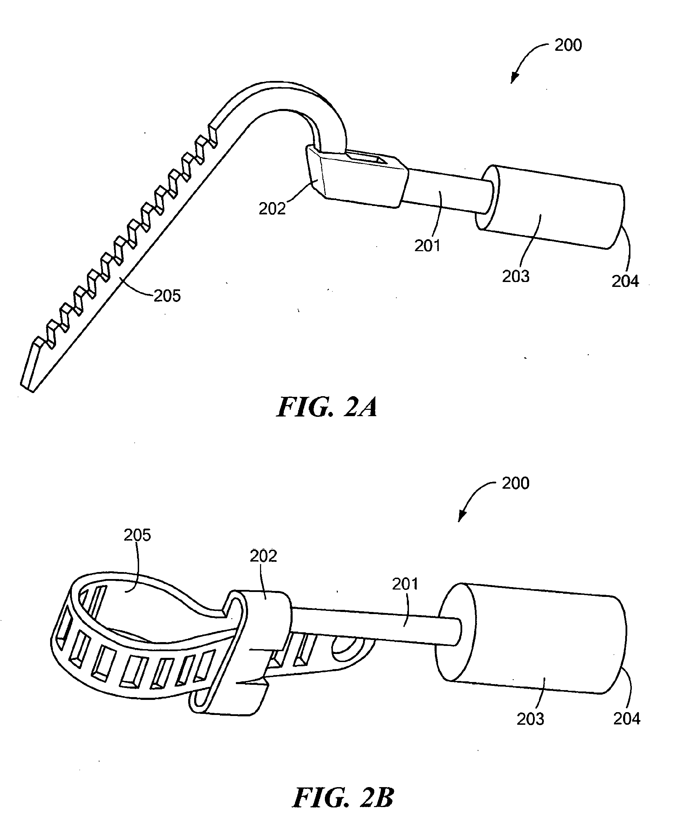

Figure 2A-B shows two examples of a PORP ossicular prosthesis **200** which includes an elongated titanium prosthesis member **201** having a proximal end and a distal end. A cochlea striker mass **203** is at the distal end of the prosthesis member **201** and includes an outer striking surface **204** for coupling vibration of the striker mass **203** to an outer cochlea surface of a recipient patient. A locking clamp is at the proximal end of the prosthesis member **201** and includes a clamp strap **205** having a fixed end and a free end, and a locking head **202** at the fixed end of the clamp strap **205.** The locking head **202** has a strap opening for insertion of the free end of the clamp strap **205.** Thus, the locking clamp is basically a cable-tie or zip-lock type clamp which because the size of loop is adjustable is a one size fits all device.

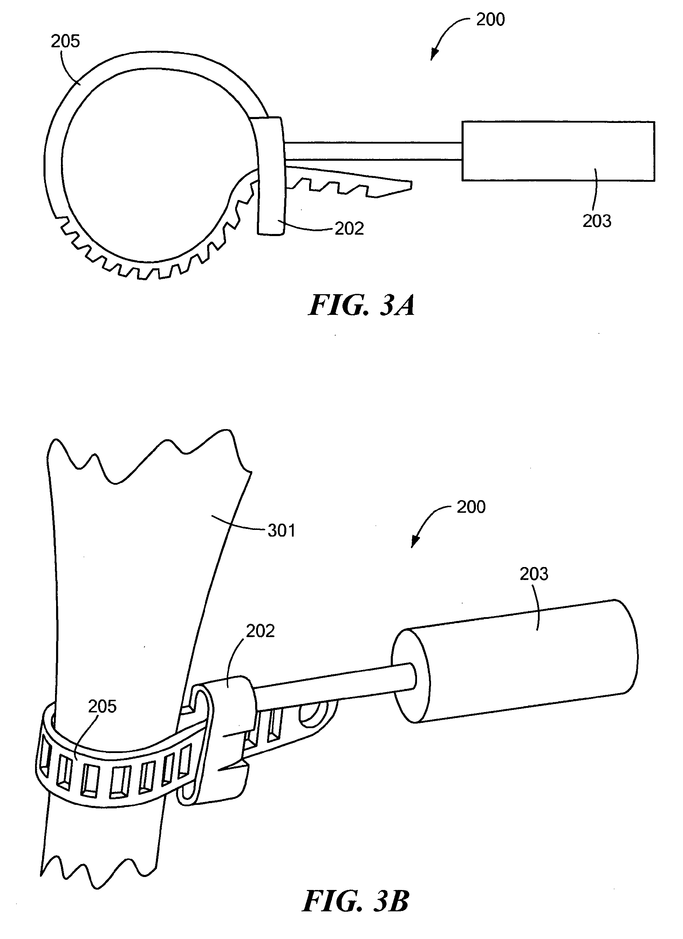

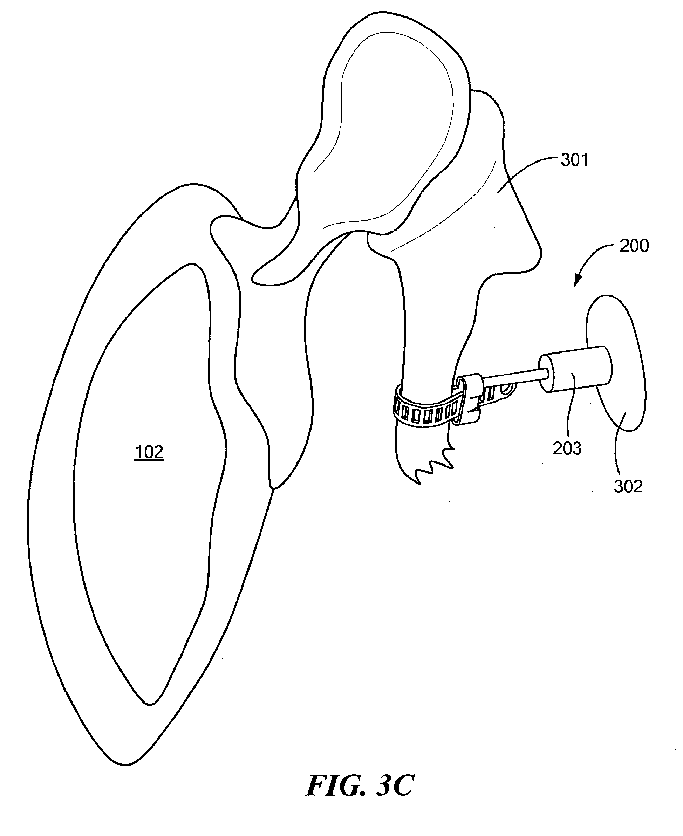

Fig. 3A shows a top view of a PROP ossicular prosthesis **200** as described above. Fig. 3B shows in close detail how the clamp strap **205** of the ossicular prosthesis **200** passes around an ossicle **301** of the middle ear (e.g., a disarticulated incus) in a closed loop and is fixedly engaged by the locking head **202** such that acoustic vibration of the ossicle **301** is coupled by the ossicular prosthesis **200** to a cochlea surface **302** of the patient such as the oval window membrane and/or the round window membrane. The clamp strap **205** is made of plastic or a polymer material so it can be tightened around the ossicle **301** and pulled snug with a desired amount of tension that can be dialed in by the surgeon. Fig. 3C shows the arrangement of Figs. 3A and 3B in situ in a recipient patient.

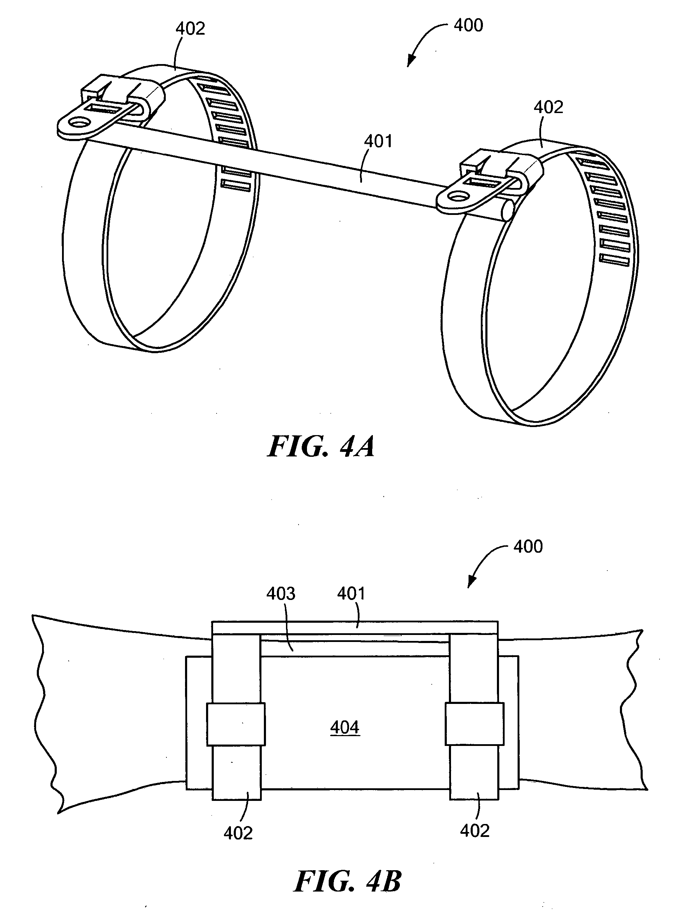

Fig. 4A-B shows another example of an ossicular connector **400** for coupling an acoustic signal transducer such as an implanted floating mass transducer (FMT) to an ossicle in the middle ear. The ossicular connector **400** includes an elongated titanium connector member **401,** at least one of which as a locking clamp **402** which includes a clamp strap and a locking head. In the embodiment shown in Fig. 4A-B, there actually is a locking clamp **402** at each end of the connector member **401.** The clamp strap of the locking clamp **402** passes around an ossicle **403** of the middle ear (e.g., a disarticulated incus) and an implanted acoustic signal transducer **404** in a closed loop for attaching the signal transducer **404** to the ossicle **403** such that acoustic vibration of the signal transducer **404** is coupled to the ossicle **403,** and/or vice versa. The locking clamp **402** may specifically be a cable tie-type clamp. The clamp strap may be made of plastic or a polymer material.

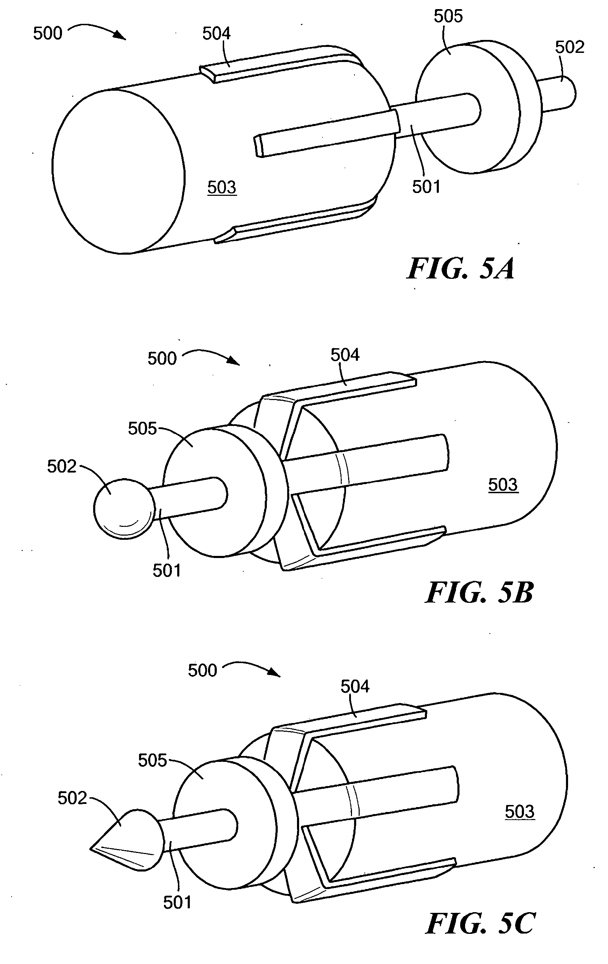

Fig. 5A-C shows other examples of a middle ear prosthesis **500** for coupling an acoustic signal transducer **503** such as an FMT to the ossicular chain, for example, an ossicular bone or the outer surface of a patient cochlea such as the oval window or round window membranes. The middle ear prosthesis **500** includes an elongated titanium prosthesis member **501** with a proximal end and a distal end. A cochlea striker mass **502** is at the distal end of the prosthesis member **501** and has an outer striking surface for coupling vibration of the striker mass **502** to the ossicular chain of a recipient patient. For example, as shown in Fig. 5A-C, the striking surface of the striker mass **502** may specifically be rod-shaped, conical, or spherical. The striker mass **502** may also include a resilient disc **505**.A transducer clamp is at the proximal end of the prosthesis member **501** and includes clamping fingers **504** for securely engaging the outer surface of an enclosed acoustic signal transducer **503** such that acoustic vibration of the signal transducer **503** is coupled by the prosthesis member **501** to the ossicular chain.

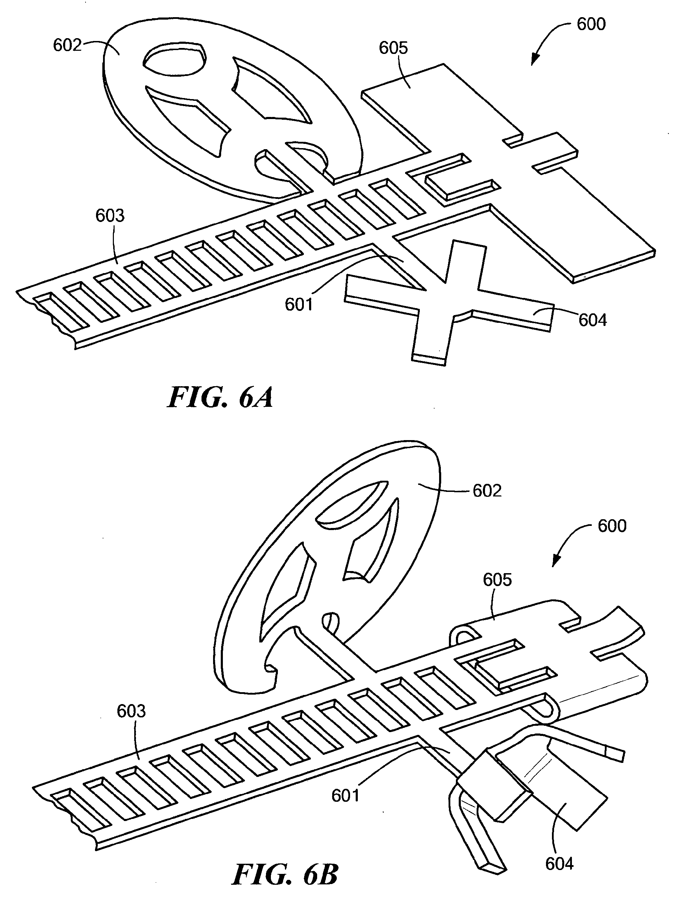

Figure 6A-B shows an embodiment of a middle ear prosthesis **600** for based a structure formed from a single folded plane. Figure 6A shows an example of a single planar sheet metal structure which is chemically etched as shown to have foldable sections **601-605.** Following the chemical etching of the metal plate into the desired shape, it may be bent with one or more manufacturing fixtures into the desired shapes as shown in Fig. 6B. In specific embodiments, all of a middle ear prosthesis **600** is formed from such a single foldable plane structure: the prosthesis member **601** and the striker mass **602,** the prosthesis member **601** and the transducer clamp **604**, or the entire prosthesis **600** is formed from a single foldable plane structure.

Embodiments of the present invention may be useful more generally in other surgeries for repair of structures where a clamp is called for, such as for limb repair other than in the middle ear. For example, a prosthesis member using a cable-tie type locking clamp to connect to a limb in some cases may avoid the need to use titanium screws. Similarly, a prosthetic strut may be implemented with a cable-tie type locking mechanism at either or both ends.

Although various exemplary embodiments of the invention have been disclosed, it should be apparent to those skilled in the art that various changes and modifications can be made which will achieve some of the advantages of the invention without departing from the true scope of the invention.

## Claims

1. A middle ear prosthesis (600) comprising:
an elongated prosthesis member (601) having a proximal end and a distal end;
a cochlea striker surface (602) at the distal end of the prosthesis member (601) for engaging an outer cochlear surface of a recipient patient; and
a clamp (604) at the proximal end of the prosthesis member (601) including a plurality of clamping fingers, **characterised in that** the clamp is a transducer clamp (604), its fingers being for securely engaging the outer surface of an enclosed acoustic signal transducer such that acoustic vibration of the signal transducer is coupled by the prosthesis member to the cochlear surface, and **in that** the prosthesis member (601) is formed from a single foldable plane structure.

2. A middle ear prosthesis (600) according to claim 1, wherein the cochlea surface includes the round window membrane of the cochlea.

3. A middle ear prosthesis (600) according to claim 1, wherein the cochlea surface includes the oval window membrane of the cochlea.

4. A middle ear prosthesis (600) according to any one of the preceding claims, wherein the prosthesis member is made of titanium.

5. A middle ear prosthesis (600) according to any one of the preceding claims, wherein the prosthesis member further comprises:
a locking clamp having:
i. a clamp strap (205 having a fixed end and a free end, and
ii. a locking head (202) at the fixed end of the clamp strap (205) and having a strap opening for insertion of the free end of the clamp strap (205);
wherein the clamp strap (205) passes around an ossicle of the middle ear in a closed loop and is fixedly engaged by the locking head (202) for fixedly attaching the prosthesis to the ossicle.

## Patentansprüche

1. Mittelohrprothese (600), aufweisend:
ein längliches Prothesenelement (601) mit einem proximalen Ende und einem distalen Ende;
eine Cochlea-Schlagfläche (602) an dem distalen Ende des Prothesenelementes (601) zum Eingriff in eine äußere Cochleafläche eines Patienten, der der Empfänger ist; und
eine Klammer (604) an dem proximalen Ende des Prothesenelementes (601), umfassend eine Vielzahl von Klemmfingern, **dadurch gekennzeichnet, dass** die Klammer eine Signalumformerklammer (604) ist, wobei deren Finger dazu ausgelegt sind, derart sicher in die Außenfläche eines eingeschlossenen akustischen Signalgebers einzugreifen, dass eine akustische Vibration des Signalgebers durch das Prothesenelement an die Cochleafläche gekoppelt wird, und dass das Prothesenelement (601) aus einer einzelnen faltbaren ebenen Struktur gebildet ist.

2. Mittelohrprothese (600) nach Anspruch 1, wobei die Cochleafläche die runde Fenstermembran der Cochlea umfasst.

3. Mittelohrprothese (600) nach Anspruch 1, wobei die Cochleafläche die ovale Fenstermembran der Cochlea umfasst.

4. Mittelohrprothese (600) nach einem der vorhergehenden Ansprüche, wobei das Prothesenelement aus Titan gefertigt ist.

5. Mittelohrprothese (600) nach einem der vorhergehenden Ansprüche, wobei das Prothesenelement ferner aufweist:
eine Sicherungsklammer mit:
i. einem Klemmriemen (205) mit einem fixierten Ende und einem freien Ende, und
ii. einem Sicherungskopf (202) an dem fixierten Ende des Klemmriemens (205) und mit einer Riemenöffnung zum Einführen des freien Endes des Klemmriemens (205);
wobei der Klemmriemen (205) in einer geschlossenen Schlaufe um ein Ossiculum des Mittelohrs geführt ist und mit dem Sicherungskopf (202) fest im Eingriff ist, um die Prothese fest an dem Ossiculum zu befestigen.

## Revendications

1. Prothèse (600) de l'oreille moyenne comprenant :
un élément (601) de prothèse allongé ayant une extrémité proximale et une extrémité distale ;
une surface (602) de percuteur pour cochlée à l'extrémité distale de l'élément (601) de prothèse pour engager une surface cochléaire extérieure d'un patient receveur ; et
une pince (604) à l'extrémité proximale de l'élément (601) de prothèse incluant une pluralité de doigts de pinçage, **caractérisée en ce que** la pince est une pince (604) de transducteur, ses doigts servent à engager de façon sûre la surface extérieure d'un transducteur intégré de signaux acoustiques, de telle sorte qu'une vibration acoustique du transducteur de signaux est couplée par l'élément de prothèse à la surface cochléaire, et **en ce que** l'élément (601) de prothèse est constitué d'une structure plane repliable unique.

2. Prothèse (600) de l'oreille moyenne selon la revendication 1, dans laquelle la surface de la cochlée inclut le tympan secondaire de la cochlée.

3. Prothèse (600) de l'oreille moyenne selon la revendication 1, dans laquelle la surface de la cochlée inclut la fenêtre vestibulaire de la cochlée.

4. Prothèse (600) de l'oreille moyenne selon l'une quelconque des revendications précédentes, dans laquelle l'élément de prothèse est constitué de titane.

5. Prothèse (600) de l'oreille moyenne selon l'une quelconque des revendications précédentes, dans laquelle l'élément de prothèse comprend en outre :
une pince de verrouillage ayant :
i. une sangle (205) de pince ayant une extrémité fixée et une extrémité libre, et
ii. une tête (202) de verrouillage à l'extrémité fixée de la sangle (205) de pince et ayant une ouverture pour sangle pour une insertion de l'extrémité libre de la sangle (205) de pince ;
dans laquelle la sangle (205) de pince passe autour d'un osselet de l'oreîlle moyenne en une boucle fermée et est engagée de façon fixe par la tête (202) de verrouillage pour fixer de façon fixe la prothèse à l'osselet.
